(19) Europäisches Patentamt
European Patent Office
Office européen des brevets

(11) Numéro de publication : **0 128 839**
**B1**

(12) # FASCICULE DE BREVET EUROPÉEN

(45) Date de publication du fascicule du brevet : **17.05.89**

(51) Int. Cl.[4] : **C 07 J 1/00**, C 07 B 59/00, A 61 K 49/02, G 01 N 33/74

(21) Numéro de dépôt : **84401202.1**

(22) Date de dépôt : **13.06.84**

(54) **Stéroides radioactifs, leur procédé et leurs intermédiaires de préparation, leur application à la mise en évidence des récepteurs de glucocorticoides et au dosage du nombre de sites de fixation.**

(30) Priorité : **14.06.83 FR 8309811**

(43) Date de publication de la demande : **19.12.84 Bulletin 84/51**

(45) Mention de la délivrance du brevet : **17.05.89 Bulletin 89/20**

(84) Etats contractants désignés : **AT BE CH DE GB IT LI LU NL SE**

(56) Documents cités :
**FR-A- 1 465 484**
**FR-A- 2 398 076**
**FR-A- 2 398 077**
**GB-A- 2 025 422**
**CHEMICAL ABSTRACTS, vol. 88, no. 9, 27 février 1978, p. 403, abrégé 62528d, Columbus, Ohio, US**

(73) Titulaire : **ROUSSEL-UCLAF**
**35, boulevard des Invalides**
**F-75007 Paris (FR)**

(72) Inventeur : **Jouquey, Alain**
**137, rue des Pyrénées**
**F-75020-Paris (FR)**
Inventeur : **Philibert, Daniel**
**16, rue Chevalier**
**F-94210-La Varenne-Saint-Hilaire (FR)**
Inventeur : **Moguilewsky, Martine**
**rue H. Tuel 80, boulevard Gambetta**
**F-94130-Nogent-Sur-Marne (FR)**
Inventeur : **Veltz, Jean-Noel**
**75, rue de Strasbourg**
**F-93200-Saint Denis (FR)**

(74) Mandataire : **Markovic, Borivoj Département des Brevets ROUSSEL UCLAF et al**
**B.P. no 9**
**F-93230 Romainville (FR)**

**EP 0 128 839 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux stéroïdes radioactifs, leur procédé et leurs intermédiaires de préparation, leur application à la mise en évidence des récepteurs de glucocorticoïdes et au dosage du nombre de sites de fixation des glucocorticoïdes.

Plus particulièrement, elle a pour objet des stéroïdes radioactifs marqués au tritium de formule générale (I) :

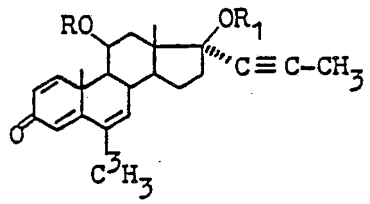

(I)

dans laquelle $^3H$ représente le tritium, R et $R_1$, semblables ou différents, représentent un atome d'hydrogène, un radical alcoyle ayant de 1 à 6 atomes de carbone ou un radical acyle ayant de 1 à 10 atomes de carbone.

Parmi les valeurs de R et $R_1$, on peut citer :

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle ;

b) les radicaux formyle, acétyle, propionyle, butyryle, isobutyryle, valéryle, caproyle, benzoyle, capryloyle, méthoxy-carbonyle ou éthoxy carbonyle.

Parmi les produits de formule générale (I), on citera notamment : la 11β, 17β-dihydroxy 6-méthyl $^3H_3$-17α-(prop-1-ynyl) androsta-1,4,6-trièn-3-one.

L'invention a également pour objet un procédé de préparation des produits répondant à la formule générale (I).

Ce procédé est caractérisé en ce que :

— on fait réagir un produit de formule générale (II) :

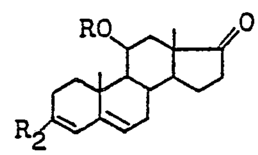

(II)

dans laquelle R a les significations précitées et $R_2$ représente un groupement alcoxy ayant de 1 à 6 atomes de carbone, un groupement acyloxy ayant de 2 à 12 atomes de carbone, ou $R_2$ représente le reste d'une amine secondaire cyclique, avec un agent d'halogéno méthylation, obtient un produit de formule générale (III) :

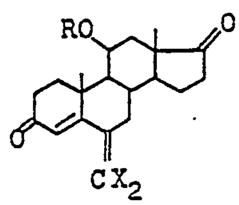

(III)

dans laquelle X représente un halogène,

— que l'on réduit par action du tritium en présence d'un catalyseur,

— bloque en milieu acide la fonction cétone en 3 du produit de formule générale (IV) résultant :

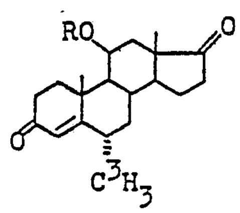

(IV)

au moyen d'un agent de O-alkylation de formule $H—C(OR_3)_3$ dans laquelle $R_3$ représente un groupement alcoyle ayant de 1 à 6 atomes de carbone, obtient un produit de formule générale (V) :

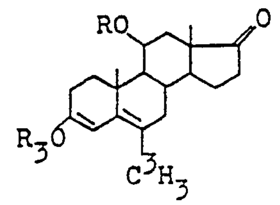

(V)

dans laquelle R et $R_3$ ont les significations précitées,

— soumet ce dernier à l'action d'un agent de propynylation,

— fait agir sur le produit de formule générale (VI) résultant :

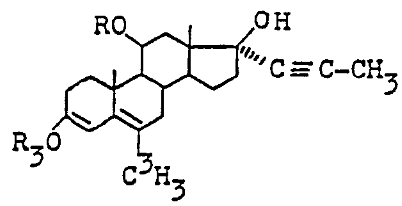

(VI)

un agent de déshydrogénation et obtient un produit de formule générale (I) dans laquelle $R_1$ représente un atome d'hydrogène que, le cas échéant,

— si R n'est pas un atome d'hydrogène, on éthérifie ou estérifie la fonction hydroxyle en 17β,

— et si R représente un atome d'hydrogène, on éthérifie ou estérifie sélectivement l'une ou l'autre des fonctions hydroxyles en 11β et 17β, ou éthérifie ou estérifie les deux fonctions hydroxyles en 11β et 17β.

Parmi les significations de $R_2$ de la formule générale (II) ci-dessus, on entend par le reste d'une amine secondaire cyclique par exemple les groupements pyrrolidino, morpholino ou pipéridino.

Dans les conditions préférentielles de mise en œuvre, le procédé est caractérisé par les points suivants :

— l'agent d'halogénométhylation est le tétrabromure de carbone et on opère sous atmosphère inerte dans le dioxanne en présence de pyridine, à température ambiante puis à température du reflux du mélange réactionnel ;

— le catalyseur en présence duquel on fait agir le tritium est l'hydroxyde de palladium et on opère dans le dioxanne en présence de triéthylamine ;

— l'agent de O-alkylation de la fonction cétone en 3 du produit de formule générale (IV) est l'orthoformiate d'éthyle et on opère sous atmosphère inerte en milieu acide ;

— l'agent de propynylation que l'on fait agir sur le produit de formule générale (V) est dans le bromure de propyne-1-yle magnésium et on opère sous atmosphère inerte dans le tétrahydrofuran ;

— l'agent de déshydrogénation que l'on fait agir sur le produit de formule générale (VI) est la dichlorodicyanoquinone et on opère sous atmosphère inerte dans un solvant aromatique ou chloré, tel que le benzène ou le chlorure de méthylène.

L'éthérification et/ou l'estérification des produits de formule générale (I) peuvent être effectuées selon les méthodes habituelles.

L'éthérification peut être effectuée par exemple au moyen d'un agent d'éthérification tel qu'un halogénure d'alcoyle ou d'aryle, ou un sulfate de dialcoyle.

L'estérification peut être effectuée par exemple au moyen d'un agent d'estérification tel qu'un dérivé fonctionnel d'acide, tel qu'un chlorure d'acide ou un anhydride.

Dans ses modes d'exécution, le procédé de l'invention peut encore être illustré par le fait que lors de l'action de l'agent d'halogénométhylation sur un produit de formule (II), il se produit successivement la condensation dudit agent sur le stéroïde à température ambiante, puis sous l'effet de la chaleur, la deshalohydratation de la molécule formée in situ.

L'invention a également pour objet l'application des produits de formule générale (I) et notamment de la 11β,17β-dihydroxy 6-méthyl $^3H_3$-17α-(prop-1-ynyl) androsta-1,4,6-trièn-3-one à la mise en évidence des récepteurs de glucocorticoïdes et au dosage du nombre de sites de fixation des glucocorticoïdes dans les tissus des organes de l'homme et des animaux.

L'invention a également pour objet le moyen pour la mise en évidence des récepteurs des glucocorticoïdes et le dosage du nombre de sites de fixation des glucocorticoïdes comportant un produit tel que défini par la formule générale (I) et notamment la 11β-17β-dihydroxy-6-méthyl-$^3H_3$-17α-(prop-1-ynyl) androsta-1,4,6-trièn-3-one.

L'invention a également pour objet à titre de produits industriels nouveaux et notamment à titre de produits intermédiaires nécessaires à l'exécution du procédé de l'invention, les produits suivants :

— la 11β-hydroxy-6-dibromométhylène-androsta-4-èn-3,17-dione ;

— la 11β-hydroxy-6-méthyl-$^3H_3$-androsta-4-èn-3,17-dione ;

— la 3-éthoxy-11β-hydroxy-6-méthyl-$^3H_3$-androsta-3,5-dièn-17-one ;

— le 3-éthoxy-11β,17β-dihydroxy-6-méthyl-$^3H_3$-17α-(prop-1-ynyl) androsta-3,5-diène.

Les produits de formule générale (I) et notamment la 11β,17β-dihydroxy-6-méthyl-$^3H_3$-17α-(prop-1-ynyl) androsta-1,4,6-trièn-3-one permettent comme déjà mentionné, la mise en évidence des récepteurs de glucocorticoïdes et le dosage du nombre de sites de fixation des glucocorticoïdes.

On savait déjà que la 11β,17β-dihydroxy-6-méthyl-17α-(prop-1-ynyl) androsta-1,4,6-trièn-3-one non radioactive se fixe d'une manière sélective sur des récepteurs de glucocorticoïdes (G. Teutsch, R. Deraedt et Al. Steroids, Vol. 38, N 6, p. 651-665, 1981).

Par contre d'autres corticoïdes naturels ou synthétiques, que l'on utilise pour ce type d'expérimentation, se fixent simultanément sur d'autres récepteurs de stéroïdes et notamment sur des récepteurs de minéralocorticoïdes.

Néanmoins, lors de telles expérimentations avec la 11β,17β-dihydroxy-6-méthyl-17α-(prop-1-ynyl) androsta-1,4,6-trièn-3-one, produit non radioactif, en particulier lorsque les tissus d'organes examinés comportaient simultanément des récepteurs de glucocorticoïdes et des récepteurs de minéralocorticoïdes, on était souvent obligé de recourir à une succession de méthodes compliquées et longues.

Or, on vient de constater à présent que les produits de formule générale (I) et notamment la 11β,17β-dihydroxy-6-méthyl-$^3H_3$-17α-(prop-1-ynyl) androsta-1,4,6-trièn-3-one, radioactive, objets de l'invention, présentent la même réactivité sélective par rapport aux récepteurs de glucocorticoïdes que les produits analogues non radioactifs.

Du fait de la radioactivité des produits de formule générale (I), il est beaucoup plus aisé de mettre en évidence l'existence des récepteurs de glucocorticoïdes dans les tissus d'organes, lorsque des récepteurs de minéralcorticoïdes sont présents également, grâce à l'application des méthodes habituelles de mesures de liaison ayant recours aux produits radioactifs.

En outre, ce qui n'était pas possible avec un produit non radioactif, comme par exemple le dosage du nombre de sites de fixation des récepteurs de glucocorticoïdes, devient réalisable grâce à l'utilisation des produits de formule (I).

On peut envisager également que lors d'une utilisation in vivo des produits de formule générale (I) et notamment de la 11β-17β-dihydroxy-6-méthyl-$^3H_3$-17α-(prop-1-ynyl) androsta-1,4,6-trièn-3-one, on puisse, grâce à leur radioactivité, déterminer aisément l'emplacement exact des sites de fixation des glucocorticoïdes.

Les produits de formule générale (I) peuvent ainsi être utilisés comme un moyen particulièrement intéressant de mise en évidence des récepteurs de glucocorticoïdes et du dosage du nombre de sites de fixation, par exemple dans les tissus des organes comportant des récepteurs des glucocorticoïdes et des récepteurs des minéralocorticoïdes, tels que l'hippocampe et autres structures du cerveau, les reins, le côlon, l'hypophyse et autres tissus pripériques.

Les produits de formule générale (I), dans des conditions de travail in vitro et dans certains tissus, ne sont pas sujet au métabolisme, dans des conditions d'incubation dans lesquelles la corticostérone ou le cortisol, par exemple, se métabolisent dans certains tissus.

Les techniques utilisées pour la mise en évidence des récepteurs de stéroïdes et le dosage du nombre de sites de fixation sont celles le plus couramment utilisées dans ce domaine :

— Raynaud J. P., Philibert D. et al., J. of Steroid Biochem., 6 (1975), 615-622 ;

— Moguilewsky et al., J. of Steroid Biochem. 12, (1980), p. 309-314.

Lors de l'expérimentation avec la 11β,17β-dihydroxy-6-méthyl-$^3H_3$-17α-(prop-1-ynyl) androsta-1,4,6-trièn-3-one (produit A), il a été constaté qu'il satisfaisait aisément à toutes les exigences nécessaires pour la mise en évidence des récepteurs de glucocorticoïdes et le dosage de sites de fixation. Il a été ainsi établi que :

a) le produit A ne donne lieu à aucune fixation spécifique sur des protéines plasmatiques chez l'animal ou chez l'homme, lesquelles protéines contaminent généralement toute préparation de récepteurs tissulaires.

b) A 0 °C la constante d'association du produit A($K_a \simeq 10^9 M^{-1}$) envers des récepteurs de glucocorticoïdes du cytosol de thymus de rat, fournie par la lecture d'adsorption au moyen de charbon-dextrane, est environ deux fois plus élevée que celle de la dexaméthasone ;

c) Le produit A est doté d'une vitesse d'association élevée ($K_{+1} \simeq 10^6 M^{-1}\ mn^{-1}$) et d'une vitesse de dissociation basse vis-à-vis des récepteurs de glucocorticoïdes dans le thymus (t1/2 ≃ 16 h) et vis-à-vis des récepteurs de glucocorticoïdes de l'hippocampe (t1/2 ≃ 24 h). Dans ces deux tissus, il se dissocie selon la courbe à une pente tandis que la $^3H$-dexamétasone et la $^3H$-corticostérone dans le cytosol d'hippocampe présentent une courbe de dissociation à deux pentes, qui est caractéristique d'une fixation sur au moins deux sites de liaison de glucocorticoïdes et de minéralocorticoïdes.

d) A 0 °C et 15 °C le produit A forme avec des récepteurs de glucocorticoïdes de thymus des complexes qui sont stables au moins 24 h.

Dans ces conditions, le dit produit A ne manifeste pas de perte de tritium ; il n'est pas sujet au métabolisme et ne présente qu'un faible fixation non spécifique.

e) Soumis à l'incubation à 15 °C pendant 24 h, le produit A remplace totalement la corticostérone non radioactive et le cortisol non radioactif fixés sur des récepteurs de glucocorticoïdes. De ce fait, il permet de doser le nombre total de sites de fixation de glucocorticoïdes dans différentes conditions physiologiques.

L'exemple suivant illustre l'invention sans toutefois la limiter.

Exemple : 11β,17β-dihydroxy 6-méthyl-$^3H_3$-17α-(prop-1-ynyl) androsta-1,4,6-trièn-3-one

Stade A : 11β-hydroxy-6-dibromométhylène-androsta-4-èn-3,17-dione

On prépare le mélange réactionnel de 3,7 g de 3-éthoxy 11β-hydroxy androsta-3,5-diène 17-one, 20 ml de dioxanne, 1 ml de pyridine et 4 g de tétrabromure de carbone, le soumet sous atmosphère inerte à l'agitation à température ambiante pendant 24 h environ, sépare le précipité, le lave à plusieurs reprises par du dioxanne, réunit les filtrats organiques et les porte au reflux pendant 4 heures environ. Une fois la solution refroidie, on ajoute 50 ml d'acide chlorhydrique 2N, soumet à l'agitation pendant une dizaine de minutes, extrait par l'acétate d'éthyle, lave les phases organiques successivement à l'eau et à l'eau saturée au chlorure de sodium, sèche et amène à sec. On reprend le résidu par l'acétate d'éthyle et laisse le produit cherché cristalliser à — 20 °C. Le produit est purifié par recristallisation dans l'acétate d'éthyle et on obtient 0,6 g de produit cherché dont le Pf = 256 °C.

Spectre UV (EtOH)
max - 252 nm
infl - 284 nm

La pureté peut être appréciée par chromatographie sur couche mince de silice en éluant par le mélange cyclohexane/acétate d'éthyle (1-1), Rf = 0,2.

Parmi les produits de départ de formule générale (II), la 11β-hydroxy-3-éthoxy-androsta-3,5-dièn-17-one a été décrite dans la demande de brevet japonais 9577/71.

D'autres produits correspondant à cette formule peuvent être préparés selon les méthodes connues en soi.

Stade B : 11β-hydroxy-6-méthyl-$^3H_3$-androsta-4-èn-3,17-dione

On refroidit par l'azote liquide le mélange réactionnel constitué par 80 mg de produit dibromé préparé au stade précédent, 2 ml de dioxanne, 20 mg de charbon palladié à 2 % sur talc et 120 µl de triéthylamine.

On introduit sous vide 15,5 ml de tritium d'une activité spécifique de 58 Ci/mmol. On laisse revenir le mélange réactionnel à température ambiante, puis chauffe vers 40 °C et agite sous tritium jusqu'à fin d'absorption. On récupère ensuite l'excès du tritium, filtre, ajoute 1 ml d'HCl 2N, abandonne au repos pendant 1 heure environ à température ambiante, extrait à l'acétate d'éthyle, lave à l'eau, sèche, évapore à sec et obtient 60 mg de produit brut, que l'on utilise tel que pour le stade suivant.

Spectre UV (EtOH)
max 240 nm

La pureté du produit peut être appréciée par chromatographie sur couche mince de silice en éluant par le mélange cyclohexane/acétate d'éthyle (1-1), Rf = 0,17.

Stade C : 3-éthoxy-11β-hydroxy-6-méthyl-$^3H_3$-androsta-3,5-dièn-17-one

On introduit sous atmosphère inerte et sous agitation le produit tritié obtenu au stade précédent dans 120 µl d'orthoformiate d'éthyle et 20 µl d'orthoformiate d'éthyle à 1 % de chlorure d'acétyle, puis ajoute 400 µl d'éthanol. On maintient l'agitation à température ambiante pendant 10 minutes environ, puis ajoute 100 µl de triéthylamine, concentre ensuite le mélange réactionnel à sec sous pression réduite et obtient 58 mg de produit brut attendu que l'on utilise tel que pour le stade suivant.

La pureté du produit peut être appréciée par chromatographie sur couche mince de silice en éluant par le mélange cyclohexane/acétate d'éthyle (1-1), Rf = 0,5.

Stade D : 3-éthoxy-11β,17β-dihydroxy-6-méthyl-$^3H_3$ 17α-(prop-1-ynyl)-androsta-3,5-diène

On introduit le produit obtenu au stade précédent, sous agitation et sous atmosphère inerte, dans 100 µl de tétrahydrofuran, ajoute 2 ml d'une solution de bromure de propyn-1-yl-magnésium 1M dans le tétrahydrofuran, maintient le mélange réactionnel sous agitation, à température ambiante, pendant une demi-heure environ, puis ajoute 5 ml d'une solution aqueuse de chlorure d'ammonium à 10 %, extrait par l'acétate d'éthyle, lave l'extrait à l'eau, le sèche et le concentre à sec. On obtient 60 mg de produit d'une activité de 7 Ci. Le produit est utilisé tel que pour le stade suivant.

La pureté du produit peut être appréciée par chromatographie sur couche mince de silice en éluant par le mélange cyclohexane/acétate d'éthyle (1-1), Rf = 0,4.

Stade E : 11β,17β-dihydroxy-6-méthyl-$^3H_3$-17α-(prop-1-ynyl) androsta-1,4,6-trièn-3-one

On ajoute en 15 minutes sous agitation et sous atmosphère inerte au produit obtenu dans le stade

précédent dans 0,5 ml de benzène, la solution de 90 mg de dichlorodicyano quinone. On maintient l'agitation pendant une demi-heure environ, puis ajoute 0,5 ml d'acétone, 100 µl de bisulfite de sodium (une solution aqueuse de d = 1,32) et 1 ml d'une solution aqueuse à 10 % de bicarbonate de soude. On extrait ensuite par l'acétate d'éthyle, lave l'extrait par une solution aqueuse à 10 % de bicarbonate de soude et obtient après purification par la chromatographie liquide haute pression sur silice en éluant par un mélange chloroforme/méthanol à 5 % d'eau (99/1), 50,3 mg de produit cherché, dont l'activité spécifique est de 37 Ci/mmol.

La chromatographie sur couche mince de silice, en éluant par le mélange cyclohexane/acétate d'éthyle (1-1) donne un produit de Rf = 0,15.

**Revendications** (pour les Etats contractants : BE, CH, DE, GB, IT, LI, LU, NL, SE)

1. Stéroïdes radioactifs de formule générale (I) :

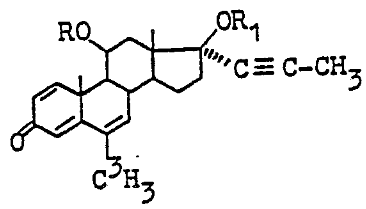

(I)

dans laquelle $^3H$ représente le tritium, R et $R_1$, semblables ou différents, représentent un atome d'hydrogène, un radical alcoyle ayant de 1 à 6 atomes de carbone ou un radical acyle ayant de 1 à 10 atomes de carbone.

2. La 11β,17β-dihydroxy 6-méthyl-$^3H_3$-17α-(prop-1-ynyl) androsta-1,4,6-trièn-3-one.

3. Procédé de préparation des produits définis aux revendications 1 et 2 caractérisé en ce que l'on fait réagir un produit de formule générale (II) :

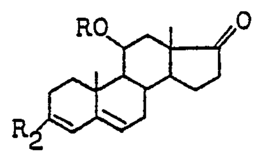

(II)

dans laquelle R a les significations indiquées à la revendication 1 et $R_2$ représente un groupement alcoxy ayant de 1 à 6 atomes de carbone, un groupement acyloxy ayant de 2 à 12 atomes de carbone, ou $R_2$ représente le reste d'une amine secondaire cyclique, avec un agent d'halogéno méthylation, obtient un produit de formule générale (III) :

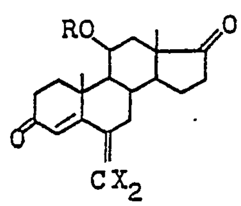

(III)

dans laquelle X représente un halogène,

— que l'on réduit par action du tritium en présence d'un catalyseur,

— bloque en milieu acide la fonction cétone en 3 du produit de formule générale (IV) résultant :

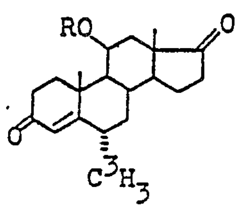

(IV)

au moyen d'un agent de O-alkylation de formule H—C($OR_3$)$_3$ dans laquelle $R_3$ représente un groupement alcoyle ayant de 1 à 6 atomes de carbone, obtient un produit de formule générale (V) :

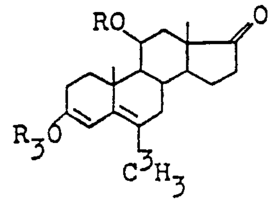

(V)

dans laquelle R et $R_3$ ont les significations précitées,

— soumet ce dernier à l'action d'un agent de propynylation,

— fait agir sur le produit de formule générale (VI) résultant :

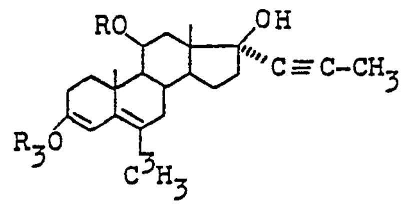

(VI)

un agent de déshydrogénation et obtient un produit de formule générale (I) dans laquelle $R_1$ représente un atome d'hydrogène que, le cas échéant,

— si R n'est pas un atome d'hydrogène, on éthérifie ou estérifie la fonction hydroxyle en 17β,

— et si R représente un atome d'hydrogène, on éthérifie ou estérifie sélectivement l'une ou l'autre des fonctions hydroxyles en 11β et 17β, ou éthérifie ou estérifie les deux fonctions hydroxyles en 11β et 17β.

4. Procédé selon la revendication 3 caractérisé en ce que l'agent d'halogénométhylation est le tétrabromure de carbone et on opère sous atmosphère inerte dans le dioxanne en présence de pyridine, à température ambiante puis à température du reflux du mélange réactionnel.

5. Procédé selon les revendications 3 et 4 caractérisé en ce que le catalyseur en présence duquel on fait agir le tritium est l'hydroxyde de palladium et on opère dans le dioxanne en présence de triéthylamine.

6. Procédé selon l'une quelconque des revendications 3 à 5 caractérisé en ce que l'agent de O-alkylation de la fonction cétone en 3 du produit de formule générale (IV) est l'orthoformiate d'éthyle et on opère sous atmosphère inerte en milieu acide.

7. Procédé selon l'une quelconque des revendications 3 à 6 caractérisé en ce que l'agent de propynylation que l'on fait agir sur le produit de formule générale (V) est le bromure de propyne-1-yle magnésium et on opère sous atmosphère inerte dans le tétrahydrofuran ;

8. Procédé selon l'une quelconque des revendications 3 à 7 caractérisé en ce que l'agent de déshydrogénation que l'on fait agir sur le produit de formule générale (VI) est la dichlorodicyanoquinone et on opère sous atmosphère inerte dans un solvant aromatique ou chloré, tel que le benzène ou le chlorure de méthylène.

9. Application des produits tels que définis par la formule générale (I) de la revendication 1 et notamment de la 11β,17β-dihydroxy 6-méthyl-$^3H_3$-17α-(prop-1-ynyl) androsta-1,4,6-trièn-3-one à la mise en évidence des récepteurs de glucocorticoïdes et au dosage du nombre de sites de fixation des glucocorticoïdes.

10. Les produits de formule générale (I) tels que définis à la revendication 1 ou 2, pour leur utilisation, chez l'homme ou l'animal, dans la mise en évidence des récepteurs des glucocorticoïdes et dans le dosage du nombre de sites de fixation des glucocorticoïdes.

11. A titre de produits industriels nouveaux :

— la 11 bêta-hydroxy-6-dibromométhylène-androsta-4-én-3, 17-dione ;

— la 11 bêta-hydroxy-6-méthyl-$^3H_3$-androsta-4-én-3,17-dione ;

— la 3-éthoxy-11 bêta-hydroxy-6-méthyl-$^3H_3$-androsta-3,5-dièn-17-one

— le 3-éthoxy-11 bêta, 17 bêta-dihydroxy-6-méthyl-$^3H_3$-17 alpha-(prop-1-nyl) androsta-3,5-diène.

**Revendications** (pour l'Etat contractant AT)

1. Procédé de préparation des stéroïdes radioactifs de formule générale (I) :

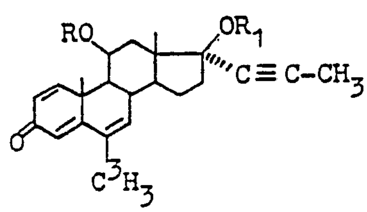

(I)

dans laquelle $^{3}H$ représente le tritium, R et $R_1$, semblables ou différents, représentent un atome d'hydrogène, un radical alcoyle ayant de 1 à 6 atomes de carbone ou un radical acyle ayant de 1 à 10 atomes de carbone, caractérisé en ce que l'on fait réagir un produit de formule générale (II) :

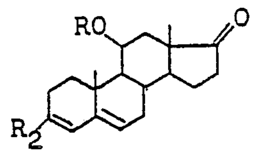

(II)

dans laquelle R a les significations indiquées précédemment et $R_2$ représente un groupement alcoxy ayant de 1 à 6 atomes de carbone, un groupement acyloxy ayant de 2 à 12 atomes de carbone, ou $R_2$ représente le reste d'une amine secondaire cyclique, avec un agent d'halogéno méthylation, obtient un produit de formule générale (III) :

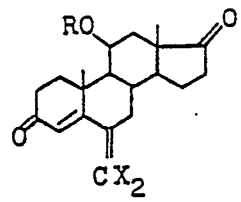

(III)

dans laquelle X représente un halogène,

— que l'on réduit par action du tritium en présence d'un catalyseur,

— bloque en milieu acide la fonction cétone en 3 du produit de formule générale (IV) résultant :

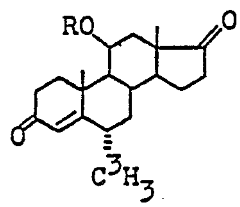

(IV)

au moyen d'un agent de O-alkylation de formule $H—C(OR_3)_3$ dans laquelle $R_3$ représente un groupement alcoyle ayant de 1 à 6 atomes de carbone, obtient un produit de formule générale (V) :

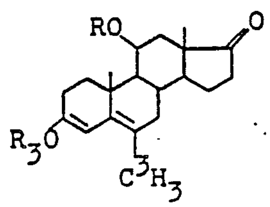

(V)

dans laquelle R et $R_3$ ont les significations précitées,

— soumet ce dernier à l'action d'un agent de propynylation,

— fait agir sur le produit de formule générale (VI) résultant :

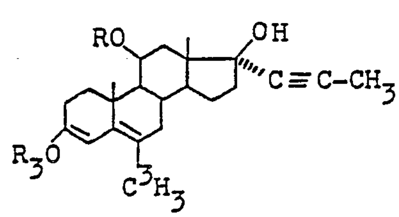

(VI)

un agent de déshydrogénation et obtient un produit de formule générale (I) dans laquelle $R_1$ représente un atome d'hydrogène que, le cas échéant,

— si R n'est pas un atome d'hydrogène, on éthérifie ou estérifie la fonction hydroxyle en 17β,

— et si R représente un atome d'hydrogène, on éthérifie ou estérifie sélectivement l'une ou l'autre des fonctions hydroxyles en 11β et 17β, ou éthérifie ou estérifie les deux fonctions hydroxyles en 11β et 17β.

2. Procédé selon la revendication 1 caractérisé en ce que l'agent d'halogénométhylation est le

tétrabromure de carbone et on opère sous atmosphère inerte dans le dioxanne en présence de pyridine, à température ambiante puis à température du reflux du mélange réactionnel.

3. Procédé selon les revendications 1 et 2 caractérisé en ce que le catalyseur en présence duquel on fait agir le tritium est l'hydroxyde de palladium et on opère dans le dioxanne en présence de triéthylamine.

4. Procédé selon l'une quelconque des revendications 1 à 3 caractérisé en ce que l'agent de O-alkylation de la fonction cétone en 3 du produit de formule générale (IV) est l'orthoformiate d'éthyle et on opère sous atmosphère inerte en milieu acide.

5. Procédé selon l'une quelconque des revendications 1 à 4 caractérisé en ce que l'agent de propynylation que l'on fait agir sur le produit de formule générale (V) est le bromure de propyne-1-yle magnésium et on opère sous atmosphère inerte dans le tétrahydrofuran ;

6. Procédé selon l'une quelconque des revendications 1 à 5 caractérisé en ce que l'agent de déshydrogénation que l'on fait agir sur le produit de formule générale (VI) est la dichlorodicyanoquinone et on opère sous atmosphère inerte dans un solvant aromatique ou chloré, tel que le benzène ou le chlorure de méthylène.

7. Procédé selon l'une quelconque des revendications 1 à 6, caractérisé en ce que l'on prépare le produit dont le nom suit. La 11 bêta, 17 bêta-dihydroxy-6-méthyl-$^3H_3$-17-alpha (prop-1-ynyl) androsta-1,4,6-trièn-3-one.

**Claims** (for the Contracting States : BE, CH, DE, GB, IT, LI, LU, NL, SE)

1. Radioactive steroids of general formula (I) :

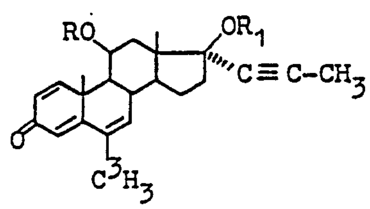

(I)

in which $^3H$ represents tritium, R and $R_1$, the same or different, represent a hydrogen atom, an alkyl radical having from 1 to 6 carbon atoms or an acyl radical having from 1 to 10 carbon atoms.

2. 11 beta, 17 beta-dihydroxy-6-methyl-$^3H_3$-17 alpha-(prop-1-ynyl)-androsta-1,4,6-trien-3-one.

3. Preparation process for products defined in claims 1 and 2, characterized in that a product of general formula (II) :

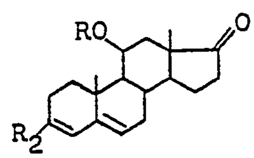

(II)

in which R has the significances indicated in claim 1 and $R_2$ represents an alkoxy group having from 1 to 6 carbon atoms, an acyloxy group having from 2 to 12 carbon atoms, or $R_2$ represents the residue of a cyclic secondary amine, is reacted with a halogeno-methylation agent, a product of general formula (III) :

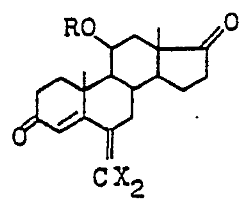

(III)

in which X represents a halogen, being obtained,

— which is reduced by the action of tritium in the presence of a catalyst,

— the ketone function in position 3 of the resulting product of general formula (IV) :

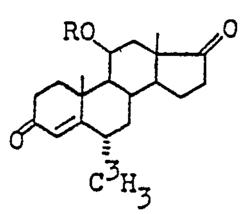

(IV)

is blocked in an acid medium by an O-alkylation agent of formula $H—C(OR_3)_3$ in which $R_3$ represents an alkyl group having from 1 to 6 carbon atoms ; a product of general formula (V) :

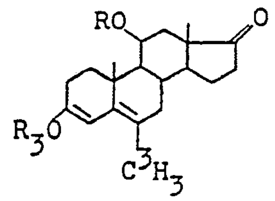

(V)

in which R and $R_3$ have the previously given significances, being obtained,

— the latter is submitted to the action of a propynylation agent,

— a dehydrogenation agent is reacted on the resulting product of general formula (VI) :

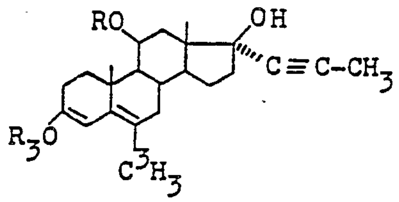

(VI)

and a product of general formula (I) is obtained, in which $R_1$ represents a hydrogen atom, of which, if appropriate,

— if R is not a hydrogen atom, the hydroxyl function in position 17 beta is etherified or esterified,

— and if R represents a hydrogen atom, one or other of the hydroxyl functions in positions 11 beta and 17 beta are selectively etherified or esterified, or both hydroxyl functions in positions 11 beta and 17 beta are etherified or esterified.

4. Process according to claim 3, characterized in that : the halogeno-methylation agent is carbon tetrabromide and the operation is carried out under inert atmosphere in dioxan in the presence of pyridine, at ambient temperature, then at the reflux temperature of the reaction mixture.

5. Process according to claims 3 and 4, characterized in that : the catalyst in the presence of which tritium is reacted is palladium hydroxide and the operation is carried out in dioxan in the presence of triethylamine.

6. Process according to any one of claims 3 to 5, characterized in that : the O-alkylation agent of the ketone function in position 3 of the product of general formula (IV) is ethyl orthoformate and the operation is carried out under inert atmosphere in an acid medium.

7. Process according to any one of claims 3 to 6, characterized in that : the propynylation agent which is reacted on the product of general formula (V) is propyne-1-yl magnesium bromide and the operation is carried out under inert atmosphere in tetrahydrofuran.

8. Process according to any one of claims 3 to 7, characterized in that the dehydrogenation agent which is reacted on the product of general formula (VI) is dichlorodicyanoquinone and the operation is carried out under inert atmosphere in an aromatic or chlorinated solvent, such as benzene or methylene chloride.

9. Use of the products as defined by general formula (I) of claim 1 and in particular of 11beta, 17beta-dihydroxy-6-methyl-$^3H_3$-17alpha-(prop-1-ynyl)androsta-1,4,6-trien-3-one, for revealing glucocorticoid receptors and for determining the number of fixation sites of glucocorticoids.

10. The products of general formula (I) as defined in claim 1 or 2, for their use, in man or animals, in revealing glucocorticoid receptors and in determining the number of fixation sites of glucocorticoids.

11. As new industrial products :

— 11beta-hydroxy-6-dibromomethylene-androsta-4-en-3,17-dione ;

— 11beta-hydroxy-6-methyl-$^3H_3$-androsta-4-en-3,17-dione ;

— 3-ethoxy-11beta-hydroxy-6-methyl-$^3H_3$-androsta-3,5-dien-17-one ;

— 3-ethoxy-11beta, 17beta-dihydroxy-6-methyl-$^3H_3$-17alpha-(prop-1-ynyl)-androsta-3,5-diene.

**Claims** (for the Contracting State AT)

1. Preparation process for radioactive steroids of general formula (I) :

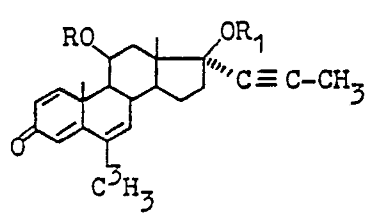

(I)

in which $^3H$ represents tritium, R and $R_1$, the same or different, represent a hydrogen atom, an alkyl radical having from 1 to 6 carbon atoms or an acyl radical having from 1 to 10 carbon atoms, characterized in that a product of general formula (II) :

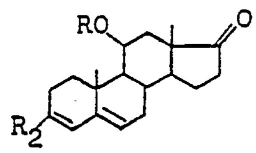

(II)

in which R has the significances indicated previously and $R_2$ represents an alkoxy group having from 1 to 6 carbon atoms, an acyloxy group having from 2 to 12 carbon atoms, or $R_2$ represents the residue of a cyclic secondary amine, is reacted with a halogeno-methylation agent, a product of general formula (III) :

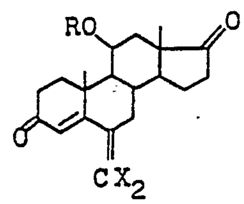

(III)

in which X represents a halogen, being obtained,

— which is reduced by the action of tritium in the presence of a catalyst,

— the ketone function in position 3 of the resulting product of general formula (IV) :

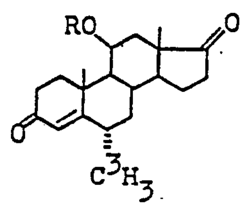

(IV)

is blocked in an acid medium by an O-alkylation agent of formula $H—C(OR_3)_3$ in which $R_3$ represents an alkyl group having from 1 to 6 carbon atoms, a product of general formula (V) :

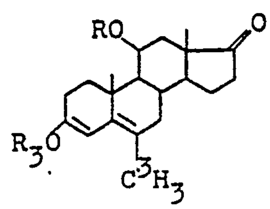

(V)

in which R and R have the previously given significances, being obtained,

— the latter is submitted to the action of a propynylation agent,

— a dehydrogenation agent is reacted on the resulting product of general formula (VI) :

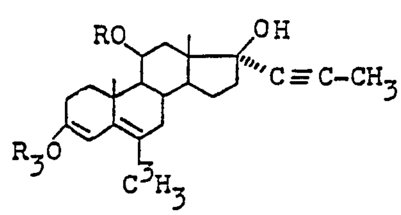

(VI)

and a product of general formula (I) is obtained, in which $R_1$ represents a hydrogen atom, of which, if appropriate,

— if R is not a hydrogen atom, the hydroxyl function in position 17beta is etherified or esterified,

— and if R represents a hydrogen atom, one or other of the hydroxyl functions in positions 11beta and 17beta are selectively etherified or esterified, or both hydroxyl functions in positions 11beta and 17beta are etherified or esterified.

2. Process according to claim 1, characterized in that : the halogeno-methylation agent is carbon

tetrabromide and the operation is carried out under inert atmosphere in dioxan in the presence of pyridine, at ambient temperature, then at the reflux temperature of the reaction mixture.

3. Process according to claims 1 and 2, characterized in that : the catalyst in the presence of which tritium is reacted is palladium hydroxide and the operation is carried out in dioxan in the presence of triethylamine.

4. Process according to any one of claims 1 to 3, characterized in that : the O-alkylation agent of the ketone function in position 3 of the product of general formula (IV) is ethyl orthoformate and the operation is carried out under inert atmosphere in an acid medium.

5. Process according to any one of claims 1 to 4, characterized in that : the propynylation agent which is reacted on the product of general formula (V) is propyne-1-yl magnesium bromide and the operation is carried out under inert atmosphere in tetrahydrofuran.

6. Process according to any one of claims 1 to 5, characterized in that the dehydrogenation agent which is reacted on the product of general formula (VI) is dichlorodicyanoquinone and the operation is carried out under inert atmosphere in an aromatic or chlorinated solvent, such as benzene or methylene chloride.

7. Process according to any one of claims 1 to 6, characterized in that the product is prepared of which the name follows : 11beta, 17beta-dihydroxy-6-methyl-$^3H_3$-17alpha-(prop-1-ynyl) androsta-1,4,6-trien-3-one.

**Patentansprüche** (für die Vertragsstaaten : BE, CH, DE, GB, IT, LI, LU, NL, SE)

1. Radioaktive Steroide der allgemeinen Formel (I)

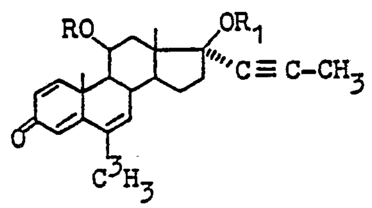

(I)

worin $^3H$ für Tritium steht, R und $R_1$, die gleich oder voneinander verschieden sind, ein Wasserstoffatom, einen Alkylrest mit 1 bis 6 Kohlenstoffatomen oder einen Acylrest mit 1 bis 10 Kohlenstoffatomen bedeuten.

2. 11β,17β-Dihydroxy-6-methyl-$^3H_3$-17α-(prop-1-inyl)-androsta-1,4,6-trien-3-on.

3. Verfahren zur Herstellung der Produkte gemäß den Ansprüchen 1 und 2, dadurch gekennzeichnet, daß man ein Produkt der allgemeinen Formel (II)

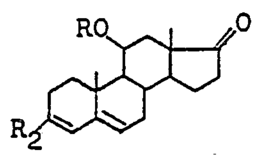

(II)

worin R die in Anspruch 1 angegebene Bedeutung besitzt und $R_2$ eine Alkoxygruppe mit 1 bis 6 Kohlenstoffatomen, eine Acyloxygruppe mit 2 bis 12 Kohlenstoffatomen wiedergibt oder $R_2$ für den Rest eines cyclischen, sekundären Amins steht, mit einem Halogenomethylierungsmittel umsetzt, um ein Produkt der allgemeinen Formel (III)

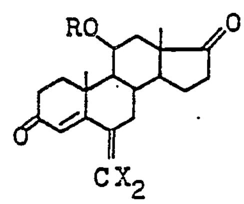

(III)

zu erlangen, worin X für ein Halogen steht,

— welches man durch Einwirken von Tritium im Gegenwart eines Katalysators reduziert,

— in saurem Milieu die Ketonfunktion in 3-Stellung des entstandenen Produkts der allgemeinen Formel (IV)

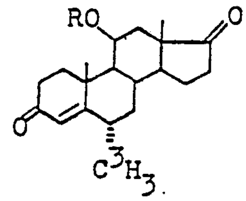

(IV)

mit Hilfe eines O-Alkylierungsmittels der Formel $H—C(OR_3)_3$, worin $R_3$ für eine Alkylgruppe mit 1 bis 6 Kohlenstoffatomen steht, blockiert, ein Produkt der allgemeinen Formel (V)

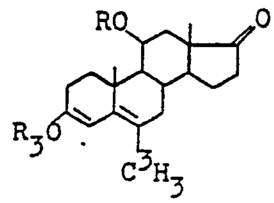

(V)

erhält, worin R und $R_3$ die vorstehenden Bedeutungen besitzen,

— dieses letztere der Einwirkung eines Propinylierungsmittels unterzieht,

— mit dem entstandenen Produkt der allgemeinen Formel (VI)

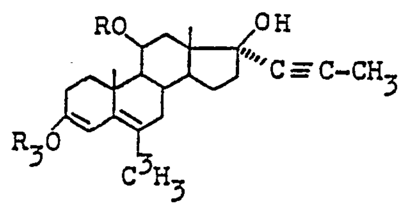

(VI)

ein Dehydrierungsmittel umsetzt und ein Produkt der allgemeinen Formel (I) gewinnt, worin $R_1$ für ein Wasserstoffatom steht, und daß man gegebenenfalls,

— wenn R nicht für ein Wasserstoffatom steht, die Hydroxylfunktion in 17β-Stellung verethert oder verestert,

— und wenn R für ein Wasserstoffatom steht, selektiv die eine oder die andere der Hydroxylfunktionen in 11β- und 17β-Stellung verethert oder verestert oder die beiden Hydroxylfunktionen in 11β- und 17β-Stellung verethert oder verestert.

4. Verfahren gemäß Anspruch 3, dadurch gekennzeichnet, daß das Halogenomethylierungsmittel Tetrabromkohlenstoff ist und man unter inerter Atmosphäre in Dioxan in Anwesenheit von Pyridin bei Raumtemperatur, dann bei Rückflußtemperatur des Reaktionsgemisches arbeitet.

5. Verfahren gemäß Anspruch 3 und 4, dadurch gekennzeichnet, daß der Katalysator, in dessen Gegenwart man das Tritium umsetzt, Palladiumhydroxid ist, und man in Dioxan in Gegenwart von Triethylamin arbeitet.

6. Verfahren gemäß einem der Ansprüche 3 bis 5, dadurch gekennzeichnet, daß das O-Alkylierungsmittel der Ketonfunktion in 3-Stellung des Produkts der allgemeinen Formel (IV) Ethylorthoformiat ist und man unter inerter Atmosphäre in saurem Milieu arbeitet.

7. Verfahren gemäß einem der Ansprüche 3 bis 6, dadurch gekennzeichnet, daß das Propinylierungsmittel, das man mit dem Produkt der allgemeinen Formel (V) umsetzt, Propin-1-yl-magnesiumbromid ist und man unter inerter Atmosphäre in Tetrahydrofuran arbeitet.

8. Verfahren gemäß einem der Ansprüche 3 bis 7, dadurch gekennzeichnet, daß das Dehydrierungsmittel, das man mit dem Produkt der allgemeinen Formel (VI) umsetzt, Dichlorodicyanochinon ist und man unter inerter Atmosphäre in einem aromatischen oder chlorierten Lösungsmittel, wie Benzol oder Methylenchlorid, arbeitet.

9. Verwendung der Produkte der allgemeinen Formel (I) gemäß Anspruch 1 und insbesondere von 11β, 17β-Dihydroxy-6-methyl-$^3H_3$-17α-(prop-1-inyl)-androsta-1,4,6-trien-3-on für die Hervorhebung der Glucocorticoidrezeptoren und die Bestimmung der Anzahl der Bindungsstellen der Glucocorticoide.

10. Die Produkte der allgemeinen Formel (I) gemäß Anspruch 1 oder 2 für deren Verwendung bei Mensch oder Tier bei der Sichtbarmachung von Glucocorticoidrezeptoren und bei der Bestimmung der Anzahl der Bindungsstellen der Glucocorticoide.

11. Als neue, industrielle Produkte das

— 11β-Hydroxy-6-dibrommethylen-androsta-4-en-3,17-dion ;

— 11β-Hydroxy-6-methyl-$^3H_3$-androsta-4-en-3,17-dion ;

— 3-Ethoxy-11β-hydroxy-6-methyl-$^3H_3$-androsta-3,5-dien-17-on ;

— 3-Ethoxy-11β,17β-dihydroxy-6-methyl-$^3H_3$-17α-(Prop-1-inyl)-androsta-3,5-dien.

**Patentansprüche** (für den Vertragsstaat AT)

1. Verfahren zur Herstellung von radioaktiven Steroiden der allgemeinen Formel (I)

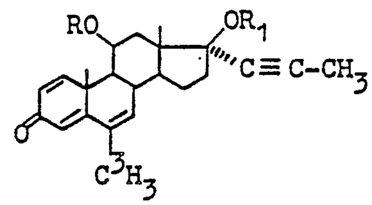

(I)

worin $^3H$ für Tritium steht, R und $R_1$, die gleich oder voneinander verschieden sind, für ein Wasserstoffatom, einen Alkylrest mit 1 bis 6 Kohlenstoffatomen oder einen Acylrest mit 1 bis 10 Kohlenstoffatomen stehen, dadurch gekennzeichnet, daß man ein Produkt der allgemeinen Formel (II)

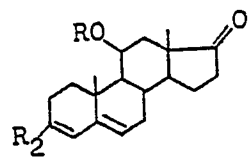

(II)

worin R die vorstehend angegebene Bedeutung besitzt und $R_2$ für eine Alkoxygruppe mit 1 bis 6 Kohlenstoffatomen, eine Acyloxygruppe mit 2 bis 12 Kohlenstoffatomen steht oder $R_2$ den Rest eines cyclischen, sekundären Amins wiedergibt, mit einem Halogenomethylierungsmittel umsetzt, um zu einem Produkt der allgemeinen Formel (III)

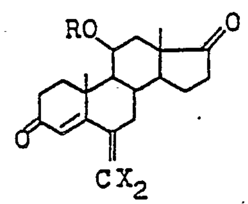

(III)

zu gelangen, worin X für ein Halogen steht,

— welches man durch Einwirken von Tritium in Gegenwart eines Katalysators reduziert,

— in saurem Milieu die Ketonfunktion in 3-Stellung des entstandenen Produkts der allgemeinen Formel (IV)

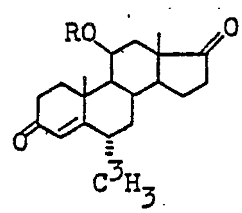

(IV)

mit Hilfe eines O-Alkylierungsmittels der Formel $H—C(OR_3)_3$, worin $R_3$ für eine Alkylgruppe mit 1 bis 6 Kohlenstoffatomen steht, blockiert, ein Produkt der allgemeinen Formel (V)

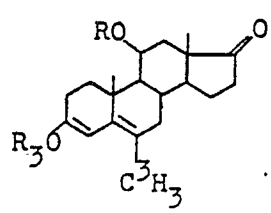

(V)

erhält, worin R und $R_3$ die vorstehenden Bedeutungen besitzen,

— dieses letztere der Einwirkung eines Propinylierungsmittels unterzieht,

— mit dem entstandenen Produkt der allgemeinen Formel (VI)

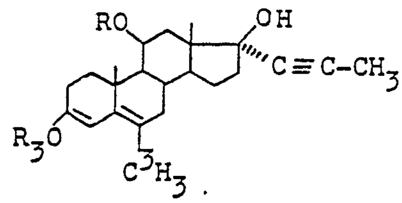

(VI)

ein Dehydrierungsmittel umsetzt und ein Produkt der allgemeinen Formel (I) gewinnt, worin $R_1$ für ein Wasserstoffatom steht, und daß man gegebenenfalls

— wenn R nicht für ein Wasserstoffatom steht, die Hydroxylfunktion in 17β-Stellung verethert oder verestert,

— und wenn R für ein Wasserstoffatom steht, man selektiv die eine oder andere der Hydroxylfunktionen in 11β- und 17β-Stellung verethert oder verestert oder die beiden Hydroxylfunktionen in 11β- und 17β-Stellung verethert oder verestert.

2. Verfahren gemäß Anspruch 1, dadurch gekennzeichnet, daß das Halogenomethylierungsmittel Tetrabromkohlenstoff ist und man unter inerter Atmosphäre in Dioxan in Anwesenheit von Pyridin bei Raumtemperatur, dann bei der Rückflußtemperatur des Reaktionsgemisches arbeitet.

3. Verfahren gemäß den Ansprüchen 1 und 2, dadurch gekennzeichnet, daß der Katalysator, in dessen Gegenwart man das Tritium umsetzt, Palladiumhydroxid ist und man in Dioxan in Anwesenheit von Triethylamin arbeitet.

4. Verfahren gemäß einem der Ansprüche 1 bis 3, dadurch gekennzeichnet, daß das O-Alkylierungsmittel der Ketonfunktion in 3-Stellung des Produkts der allgemeinen Formel (IV) Ethylorthoformiat ist und man unter inerter Atmosphäre in saurem Milieu arbeitet.

5. Verfahren gemäß einem der Ansprüche 1 bis 4, dadurch gekennzeichnet, daß das Propinylierungsmittel, das man mit dem Produkt der allgemeinen Formel (V) umsetzt, das Propin-1-ylmagnesiumbromid ist und daß man unter inerter Atmosphäre in Tetrahydrofuran arbeitet.

6. Verfahren gemäß einem der Ansprüche 1 bis 5, dadurch gekennzeichnet, daß das Dehydrierungsmittel, das man mit dem Produkt der allgemeinen Formel (VI) umsetzt, das Dichlorodicyanochinon ist und daß man unter inerter Atmosphäre in einem aromatischen oder chlorierten Lösungsmittel, wie Benzol oder Methylenchlorid, arbeitet.

7. Verfahren gemäß einem der Ansprüche 1 bis 6, dadurch gekennzeichnet, daß man das Produkt mit der folgenden Bezeichnung herstellt: 11β,17β-Dihydroxy-6-methyl-$^3H_3$-17α-(prop-1-inyl)-androsta-1,4,6-trien-3-on.